# EUROPÄISCHE PATENTSCHRIFT

(11) **EP 2 493 453 B1**
(45) Veröffentlichungstag und Bekanntmachung des Hinweises auf die Patenterteilung: **13.07.2022**
(21) Anmeldenummer: 10768958.0
(22) Anmeldetag: 26.10.2010
(51) Int. Cl.: A61L 27/22, A61L 27/34, A61L 27/52, A61K 9/06, A61K 47/42, A61K 31/00, A61K 35/30, A61K 35/32, A61K 38/38, A61K 9/00, A61L 27/54, A61K 47/64, A61P 3/04, A61P 9/00, A61P 9/12, A61P 11/06, A61P 17/06, A61P 19/04, A61P 27/02, A61P 31/00, A61P 37/00

(54) **THERAPEUTISCHE VERWENDUNG VON BIOKOMPATIBLEN ZUSAMMENSETZUNGEN UND HIERAUS POLYMERISIERTEN MATERIALIEN ZUR INHIBIERUNG DER ANGIOGENESE**
THERAPEUTIC USE OF BIOCOMPATIBLE COMPOSITIONS AND MATERIALS POLYMERIZED THEREFROM FOR INHIBITING ANGIOGENESIS
UTILISATION THERAPEUTIQUE DE COMPOSITIONS BIOCOMPATIBLES ET DE MATIÈRES POLYMÉRISÉES À PARTIR DE CES DERNIÈRES POUR INHIBER L'ANGIOGENÈSE

(30) Priorität: 26.10.2009 DE 102009051575
(43) Veröffentlichungstag der Anmeldung: 05.09.2012
(73) Patentinhaber: NMI Naturwissenschaftliches und Medizinisches Institut an der Universität Tübingen, 72770 Reutlingen (DE)
(72) Erfinder: MOLLENHAUER, Juergen, 72764 Reutlingen (DE); SCHLOSSHAUER, Burkhard, 72074 Tuebingen (DE); SCHOLZ, Beate, 69221 Dossenheim (DE)
(74) Vertreter: Witte, Weller & Partner Patentanwälte mbB
(86) Internationale Anmeldenummer: PCT/EP2010/066158
(87) Internationale Veröffentlichungsnummer: WO 2011/054699

(56) Entgegenhaltungen:
- WO-A1-2009/010232
- WO-A2-01/60335
- DE-A1-102008 008 071

## Beschreibung

Die vorliegende Erfindung betrifft die therapeutische Verwendung einer biokompatiblen Zusammensetzung und eines hieraus polymerisierten Materials, basierend auf vernetzbarem hydrophilen Polymer, zur Inhibierung und/oder Prävention der Angiogenese.

Als Angiogenese bezeichnet man die Ausbildung von neuen Gefäßstrukturen, die eine Endothelzell-Auskleidung sowie auch glatte Muskelzellen und Perizyten aufweisen. Die Angiogenese spielt bei sowohl physiologischen Prozessen, bspw. in der Embryonalentwicklung und der Wundheilung, als auch bei pathologischen Prozessen, bspw. bei Polyarthritis und Tumorwachstum, eine große Rolle.

In der Forschung und Literatur wurden und werden für die Neubildung von Gefäßen zum Teil drei unterschiedliche Begriffe verwendet - Vaskulogenese, Angiogenese Arteriogenese - wobei sich heute der Begriff Angiogenese als Überbegriff für alle Formen der Gefäßneubildung durchgesetzt hat, da eine Abgrenzung der drei genannten Formen teilweise schwierig und das zugrunde liegende Prinzip einheitlich ist.

Bei der Angiogenese handelt es sich um einen komplexen Prozess, bei dem die zur Bildung der Gefäßwände notwendigen Endothelzellen, Perizyten, und glatten Muskelzellen durch verschiedene angiogenetische Wachstumsfaktoren, bspw. durch den Fibroblasten-Wachstumsfaktor "Fibroblast Growth Factor (FGF)" und/oder den vaskulären endothelialen Wachstumsfaktor "Vascular Endothelial Growth Factor (VEGF)", aktiviert werden. Neue Kapillaren entstehen durch Proliferation und Migration von im betreffenden Gewebe bereits vorliegenden Endothelzellen.

Die Angiogenese ist von erheblicher biologischer und medizinischer Bedeutung, wobei zwei therapeutische Anwendungen der Angiogenese unterschieden werden, die Pro-angiogenetische Behandlung und die anti-angiogenetische bzw. nonangiogenetische Behandlung.

Bei ersterer soll die Gefäßneubildung stimuliert werden, insbesondere durch Einsatz und Verabreichung von Wachstumsfaktoren, wie bspw. zur Behandlung der Arteriosklerose, insbesondere der koronaren Herzkrankheit und der peripheren Verschlusskrankheit.

Eine anti- bzw. non-angiogene Behandlung wird insbesondere dort eingesetzt, wo eine Gefäßneubildung unbedingt verhindert und unerwünscht ist, wie bspw. bei der Tumorbehandlung, da solide Tumoren abhängig von einem mitwachsenden Kapillarnetz sind, das den Tumor mit Sauerstoff und Nährstoffen versorgt. Entsprechend versuchen anti-angiogenetische Therapieansätze die Gefäßversorgung und damit die Durchblutung eines Tumors zu reduzieren/zu blockieren. So wurden für eine antianiogenetische Behandlung von Tumoren im Stand der Technik bspw. VEGFneutralisierende monoklonale Antikörper eingesetzt.

Auch bei anderen Krankheiten, wie Morbus Crohn, Psoriasis, und rheumatoide Arthritis, spielt eine ungehinderte Angiogenese eine große Rolle, da über die neu gebildeten Gefäße ein ständiger Zufluss für entzündliche Zellpopulationen an die betroffenen Stellen im Körper geschaffen wird. Eine Übersicht über Krankheiten und Erkrankungen, die mit einer Angiogenese in einem unmittelbaren Zusammenhang stehen, findet sich bspw. in der Tabelle 1 in der Veröffentlichung von Polverini, "Angiogenesis in health and Disease: Insights into Basic Mechanisms and Therapeutic Opportunities", Journal of Dental Education, (2002) Vol. 66, 962-975.

Auch bei implantierbaren medizinischen Vorrichtungen/Implantaten, also bspw. Implantate, mit denen beschädigtes Gewebe ersetzt werden soll, oder Stent/Stentgrafts, die in bestimmte Organe eingebracht werden, um deren Wand abzustützen, ist oftmals Voraussetzung für einen dauerhaft erfolgreichen Einsatz, dass diese nicht die Gefäßneubildung an der Stelle, an der sie implantiert wurden, fördern, sondern sich möglichst neutral und inert in das sie umgebende Gewebe einfügen, wo sie ggf. auch resorbiert werden. In diesen Fällen besteht, bei medizinischen Implantaten die große Gefahr, dass sich Endothelzellen an diese anheften und dadurch die Mechanismen der Gefäßneubildung in Gang setzen. Dadurch können unerwünschte Nebenwirkungen, wie bspw. Schwellungen und Verdickungen des Gewebes, in das die Vorrichtung implantiert wurde, bis hin zum Tumorwachstum, auftreten.

Um dies zu verhindern, werden im Stand der Technik die zu implantierenden Vorrichtungen oftmals mit anti-angiogenen (und auch entzündungshemmenden) Wirkstoffen beschichtet, wie bspw. Antikörper (bspw. anti-VEGF Antikörper), Retinonsäure und ihre Derivate, Suramin, Metallproteinasen-1- und Metallproteinasen-2-Inhibitoren, Epothilon, Colchicin, Vinblastin, Paclitaxel, etc., die die Adhäsion von Endothelzellen an die Vorrichtungen, und die dadurch triggerbare Gefäßneubildung, inhibieren sollen.

Derartig beschichtete Vorrichtungen/Implantate haben allerdings den Nachteil, dass einerseits die Herstellung aufgrund des zusätzlichen Beschichtungsschrittes aufwändig ist, und dass andererseits die anti- oder non-angiogene Wirkung der Beschichtung von der Qualität/Quantität der Anbringung der Beschichtung und des Wirkstoffes sowie von der Dauerhaftigkeit der Beschichtung abhängt. Ferner zeigte sich in der Vergangenheit, dass selbst beschichtete Implantate die Adhäsion von Endothelzellen nicht vollständig verhindern konnten. Darüber hinaus verursachen die Beschichtungen oftmals Nebenwirkungen im zu behandelnden Patienten, die nicht nur den Erfolg des jeweiligen Eingriffs beeinflussen, solchen auch insgesamt gesundheitsschädlich sein können.

Es besteht daher gegenwärtig nach wie vor ein großer Bedarf an der Bereitstellung von medizinischen Implantaten, mit welchen die Nachteile des Standes der Technik überwunden werden können, und die - bei gleichzeitiger hervorragender Biokompatibilität - effizient im Einsatz und kostengünstig in der Herstellung sind.

Vor diesem Hintergrund ist es Aufgabe der vorliegenden Erfindung, neue Mittel für ein medizinisches Implantat bereitzustellen, die keine Gefäßneubildung auslösen bzw. verursachen, bzw. die die Adhäsion von Endothelzellen inhibieren. Erfindungsgemäß wird die Aufgabe durch die biokompatiblen Zusammensetzung, die zu einem therapeutische hydrogelbildenden Material polymerisierbar ist und die auf einem hydrophilen Polymer basiert, wobei das hydrophile Polymer vernetzbares Serumalbumin oder vernetzbares Serumprotein ist, zur therapeutischen Verwendung in der Inhibierung und/oder Prävention der Angiogenese oder der Endothelzell-Proliferation.

Ferner betrifft die Erfindung auch die therapeutische Verwendung der genannten biokompatiblen Zusammensetzung zur Beschichtung und Oberflächenmodifikation von Implantaten, die aus anderen Materialien als das Material bestehen, das ausgehend von der genannten Zusammensetzung polymerisiert ist.

Die Aufgabe wird ferner durch die biokompatible Zusammensetzung eines polymerisierten, hydrogelbildenden Materials gelöst, das durch Polymerisierung einer auf Serumalbumin oder Serumproteinen basierenden Zusammensetzung erhalten wurde, zur therapeutischen Verwendung in der Inhibierung und/oder Prävention der Angiogenese oder Endothelzell-Proliferation.

Die der Erfindung zugrunde liegende Aufgabe wird dadurch vollkommen gelöst.

Mit der erfindungsgemäßen therapeutische Verwendung der Zusammensetzung und dem polymerisierten, hydrogelbildenden Material wird ein neues therapeutisches Mittel bzw. ein medizinisches Trägermaterial bereitgestellt, das die Möglichkeit bspw. zum Gewebeersatz mittels eines Implantats ermöglicht und gleichzeitig die Adhäsion und Proliferation von Endothelzellen daran inhibiert. Vorteilhafterweise werden dadurch die Gefäßneubildung sowie eine Schwellung und Verdickung des Gewebes, in das die Zusammensetzung zum Ersatz eines erkrankten oder defizienten Gewebes eingebracht wird, vermieden, und gleichzeitig das defiziente oder erkrankten Gewebe durch Resorption des Materials ersetzt.

Mit der erfindungsgemäßen therapeutische Verwendung wird also ein Trägermaterial für ein Implantat bereitgestellt, mit dem die Angiogenese gezielt inhibiert werden kann, und bspw. das Wachstum von anderen Zellen, die nicht an der Angiogenese beteiligt sind, durch vorherige Einbringung in die Zusammensetzung/das Material gezielt gefördert werden kann.

Vorteilhaft an der neuen therapeutischen Verwendung ist darüber hinaus, dass die Zusammensetzung auch erst *in situ* polymerisierbar ist, d.h. die Zusammensetzung kann an die Stelle injiziert werden, wo der Gewebeersatz bzw. die Gewebeunterstützung stattfinden soll, und polymerisiert dann erst an dieser Stelle aus. Dadurch ist für die beanspruchte therapeutische Behandlung lediglich ein minimaler medizinischer Eingriff notwendig. Andererseits kann die Zusammensetzung auch vor Einbringung in den Körper eines Patienten auspolymerisiert werden, und anschließend über einen chirurgischen Eingriff implantiert werden.

Die Erfinder haben in eigenen Versuchen gezeigt, dass eine auf Serumalbumin bzw. Serumproteine basierende Zusammensetzung bzw. das hieraus polymerisierte Material als Trägermaterial für die Inhibierung der Adhäsion von Endothelzellen und damit für die Inhibierung/Prävention der Angiogenese hervorragend geeignet sind. So kann die auf Serumalbumin/-proteinen basierende Zusammensetzung/das Material als zu medizinisches Implantat zur Inhibierung der Angiogenese insbesondere dann eingesetzt werden, wo eine Gefäßneubildung von Nachteil ist und/oder unbedingt verhindert werden muss, bspw. bei einem Gewebeersatz von Knorpel, Bandscheiben, Cornea. Überraschenderweise hat sich in den der Erfindung zugrunde liegenden Versuchen gezeigt, dass im Vergleich zu anderen, im Stand der Technik verwendeten und bekannten Trägern oder Matrices, nur das auf Serumalbumin/-proteinen basierende Material eine Adhäsion von Endothelzellen inhibiert. Dabei ist das Material aber für die Endothelzellen selber nicht toxisch - und damit auch nicht für den Patienten, der das Material als medizinisches Implantat erhalten soll-, was wiederum die besonders hohe Bioverträglichkeit des Materials für den Patienten beweist. Darüber hinaus können Serumalbumine eine große Anzahl unterschiedlicher Substanzen, wie z.B. Metallionen (Metalle), Fettsäuren und Aminosäuren, verschiedene Proteine und Arzneimittel binden, weshalb sie äußerst biokompatibel sind und so gut wie keine Reaktionen im Körper hervorrufen.

Daher kann die erfindungsgemäße therapeutische Verwendung auch in Kombination mit anderen biologisch und/oder therapeutische aktiven Substanzen erfolgen, die über die Zusammensetzung bzw. das Gel am Zielort des Patienten eine biologische und/oder therapeutische Wirkung entfalten sollen. Dabei kann die erfindungsgemäße Verwendung derart erfolgen, dass das Material erst *in situ* polymerisiert oder aber vor dem Implantierung bereits polymerisiert ist, und im Hydrogel-Zustand implantiert wird. Dabei versteht es sich, dass bei einer Implantation des auspolymerisierten Hydrogels eine eher festere Konsistenz des Hydrogels bevorzugt ist, die eine praktikable Handhabung des Hydrogels ermöglicht bzw. erleichtert. Der Grad des Festigkeit, bzw. die Fluideigenschaft des Hydrogels bzw. des Material kann dabei über dessen Vernetzungsgrad eingestellt werden, wonach des Hydrogel bzw. das Material um so fester ist, je stärker vernetzt es ist. Die Fluideigenschaften eines Gels liegen somit zwischen der einer Flüssigkeit und der eines Feststoffkörpers.

Obgleich Albumin als bioverträgliche Substanz bekannt ist, und auch als Gel bzw. Trägermaterial als solches bspw. in der DE 10 2008 008 071.3 beschrieben ist, so war dessen Verwendung zur Inihibierung der Adhäsion und Proliferation von Endothelzellen, sowie zur Inhibierung der Angiogenese, nicht bekannt.

Die für die erfindungsgemäße therapeutische Verwendung einzusetzende Zusammensetzung bzw. das hierauf basierende polymerisierte hydrogelbildende Material kann dabei Serumalbumin/Serumproteine aufweisen, die von jedem Säugetier gewonnen werden, bzw. entsprechend für jedes Säugetier eingesetzt werden können, wobei humanes, Rinder-, Schaf- Kaninchenserumalbumin bevorzugt sind, und wobei die erfindungsgemäße Verwendung vorzugsweise beim Menschen mit einem auf humanem Serumalbumin basierenden Material eingesetzt wird.

Vorteilhaft bei der erfindungsgemäßen therapeutische Verwendung ist ferner, dass die Vorstufe des hydrogelbildenden Materials bei Raumtemperatur gehandhabt werden kann. Das Material kann demnach separat von den jeweils einzubringen Zusätzen oder Zellen gelagert werden und kurz vor der erfindungsgemäßen Anwendung mit den Zusätzen, falls erwünscht, oder ggf. Zellen, die bspw. Gewebeneubildung unterstützen sollen, zusammengeführt werden. Die Polymerisationszeit ist dabei einstellbar, wobei Zeiten zwischen wenigen Sekunden und 2 Minuten vorgesehen sein können. Daher werden die Zusätze und/oder Zellen sofort in dem Material verankert, so dass eine unerwünschte Diffusion aus dem Material vermieden wird. Dabei kann die Anwendung, wie bereits zuvor erwähnt, entweder mit dem *in situ* polymerisierbaren hydrogelbildenden Material erfolgen, oder aber mit einem vor der Einbringung in den Körper eines Patienten bereits zu einem Hydrogel-Material polymerisiert sein.

In der vorliegenden Anmeldung werden bei der erfindungsgemäßen therapeutische Verwendung die Begriffe "Zusammensetzung" und "Material" verwendet, wobei "Zusammensetzung" überwiegend, aber nicht ausschließlich, für das noch nicht polymerisierte Material verwendet wird, und "Material" oder "Gel" für die polymerisierte Zusammensetzung. Gleichwohl versteht es sich, dass diese Begriffe nicht vollständig voneinander getrennt werden können, da die Zusammensetzung und das Material faktisch den gleichen Gegenstand meinen. Dabei wird unter "Gel" der halbfeste Zustand der Zusammensetzung verstanden, der in Form eines dreidimensionalen, polymerisierten Netzwerks vorliegt.

Vorteilhaft ist bei der erfindungsgemäßen therapeutische Verwendung ferner, dass der Grundstoff für das hydrophile Polymer variabel ist, so dass einerseits kommerziell verfügbares Albumin, bspw. humanes Albumin, gereinigt oder rekombinant hergestellt, eingesetzt werden kann, sowie auch allogenes oder autologes Serum.

Wie bereits erwähnt, erfolgt die erfindungsgemäße therapeutische Verwendung derart, dass die auf Serumalbumin bzw. Serumprotein basierende Zusammensetzung in die zu behandelnde Stelle injiziert wird, wo sie zu dem hydrogelbildenden Material polymerisiert, oder das polymerisierte hydrogelbildende Material wird direkt implantiert. Nach der Einbringung in den Patienten löst sich das vernetzte Albumin innerhalb eines bestimmten Zeitraumes auf, währenddessen bspw. in dem Material vorliegende Zellen *in situ* eine perizelluläre Matrix entwickelt haben, und so in die Umgebung eingenistet werden. Gleichzeitig wird verhindert, dass sich Endothelzellen anheften und proliferieren und so eine Gefäßneubildung ausgehend von dem Material triggern.

Dabei ist in einer Ausführungsform der erfindungsgemäßen therapeutische Verwendung vorgesehen, wenn die Albuminkonzentration im polymerisierten hydrogelbildenden Material von zwischen ca. 5 bis ca. 20, insbesondere ca. 10 mg/ml Material beträgt.

Beispielhafte Verfahren zum Herstellen der Zusammensetzung für die erfindungsgemäße therapeutische Verwendung finden sich in der bereits oben erwähnten DE 10 2008 008 071.3, auf deren Inhalt diesbezüglich hiermit explizit Bezug genommen wird.

Erfindungsgemäß ist in einer bevorzugten Ausführungsform vorgesehen, dass bspw. lebende Säugetierzellen, insbesondere humane lebende Zellen, sowie ein pharmakologisches Agens, ein biologisch wirksames Agens, oder eins oder mehrere oder Mischungen davon zusammen mit der Zusammensetzung/dem Material verwendet werden.

Unter Säugetierzellen wird dabei jede Zelle verstanden, die von einem Säugetier abgeleitet ist bzw. abstammt, wobei hierunter insbesondere humane und tierische Zellen fallen. Solche Zellen können bspw. ausgewählt sein unter muskuloskelettäre Zellen, insbesondere Chondrocyten, Osteocyten, Fibrochondrocyten, sowie stoffwechselregulierende Drüsenzellen, Inselzellen, Melatonin-produzierende Zellen, Vorläuferzellen und Stammzellen, insbesondere mesenchymalen Stammzellen, mithin also Zellen, die für den jeweiligen Einsatz der Zusammensetzung, bzw. für die jeweilige Injektionsstelle geeignet und gewünscht sind. Diese Zellen sind in der Zusammensetzung bzw. dem polymerisierten hydrogelbildenden Material lebensfähig und entwickeln ein neues Gewebe bei gleichzeitiger Resorption des Materials.

Die erfindungsgemäße therapeutische Verwendung ist auch geeignet, um eine Gefäßneubildung bei Therapien zu verhindern, die das Ziel der *in situ*-Hormonproduktion haben, wie bspw. Insulin, Thyroxin oder Melatonin. Wenn Zellen, die diese oder andere Hormone produzieren, über die Zusammensetzung oder das Material an die zu behandelnden Stelle im Körper eines Patienten eingebracht werden, produzieren diese die jeweiligen Hormone und setzen diese an die Umgebung frei, wobei gleichzeitig eine Gefäßneubildung verhindert wird.

Es versteht sich, dass die erfindungsgemäße therapeutische Verwendung auch in Zusammenwirkung mit biologischen oder pharmazeutisch aktiven Substanzen erfolgen kann. "Biologisch aktive bzw. wirksame Substanz" und "pharmazeutisch aktive bzw. wirksame Substanz" soll dabei jede natürliche oder synthetische Substanz bedeuten, die entweder einen biologischen oder pharmazeutischen Einfluss auf Zellen oder Gewebe haben kann, bzw. die Reaktionen auf oder in Zellen ausüben kann. Dieser Einfluss kann dabei auf bestimmte Zellen und bestimmte Bedingungen beschränkt sein, ohne dass die Substanz ihre biologisch oder pharmazeutische aktive Bedeutung verlieren würde. Die chemische Beschaffenheit der vorliegend verwendbaren Substanzen ist dabei nicht auf eine bestimmte (Verbindungs-)Klasse beschränkt, sondern kann vielmehr jede natürliche und synthetische Substanz mit einschließen, die von ihrer Natur aus und/oder in modifizierter Form irgendeine Wirkung auf biologische Zellen ausübt.

So ist insbesondere bevorzugt, wenn als biologisch oder pharmazeutisch aktive bzw. wirksame Substanzen bspw. Antibiotika, entzündungshemmende Mittel, Stoffwechsel-Hormone, Chondroprotektiva, Agentien zur Gentherapie, Wachstumshormone oder Differenzierungs- und/oder Modulationsfaktoren, Immunsuppresiva, immun-stimulierende Substanzen, allgemein Peptide, Proteine, Nukleinsäuren, organische Wirkstoffe, Hyaluronsäure, Apoptose-induzierende Wirkstoffe, Rezeptoragonisten- und Rezeptorantagonisten, oder Mischungen davon, eingesetzt werden. Ferner können Proteine der extrazellulären Matrix, Proteine der Zelloberfläche, sowie allgemein Polysaccharide, Lipide, Antikörper, Wachstumsfaktoren, Zucker, Lektine, Kohlenhydrate, Zytokine, DNA, RNA, siRNA, Aptamere, sowie bindungs- oder wirkungsrelevante Fragmente davon, sowie sogenannte Disease-modifiying Osteoarthritis Agents, oder Mischungen davon, eingesetzt werden. Dabei können sämtliche Substanzen synthetisch hergestellt oder natürlich vorkommend sein bzw. aus rekombinanten Quellen stammen. Unter "Disease-modifying Osteoarthritis Agents" (DMOAs) sind dabei eine Reihe von Substanzen zu verstehen, die als Medikament gegenwärtig insbesondere bei Arthrose - inzwischen aber auch bei weiteren Autoimmunkrankheiten - zur Linderung von Schmerzen und Entzündungen eingesetzt werden, und deren exakter Wirkungsmechanismus bisher noch nicht umfassend geklärt ist. Die meisten dieser Substanzen enthalten Mischungen aus Glucosamin und Chondroitin-Sulfat.

Insbesondere ist in einer Ausführungsform der erfindungsgemäßen therapeutische Verwendung bevorzugt, wenn die biologisch wirksame Substanz Hyaluronsäure ist und in dem Material in einer Endkonzentration von zwischen ca. 1 bis ca. 10 mg/ml Material vorliegt, insbesondere mit a. 4 mg/ml Material.

Weitere Beispiele schließen, jedoch nicht ausschließlich, die folgenden synthetischen oder natürlichen oder rekombinanten Quellen davon mit ein: Wachstumshormone, einschließlich humanem Wachstumshormon und rekombinatem Wachstumshormon (rhGH), Rinderwachstumshormone, Schweinewachstumshormone; Wachstumshormon-freisetzende Hormone; Interferone, einschließlich Interferon-alpha, - beta und -gamma; Interleukin-1; Interleukin-2; Insulin; Insulin-ähnlicher Wachstumsfaktor, einschließlich IGF-1; Heparin; Erythropoietin; Somatostatin; Somatotropin; Proteaseinhibitoren; Adrenocorticotropin; Prostaglandine; sowie Analoga, Fragmente, Mimetika oder Polyethyleneglycol(PEG)-modifizierte Derivate dieser Verbindungen; oder eine Kombination davon. Es versteht sich, dass alle gegenwärtig im allgemeinen Gebiet der Therapie von Erkrankungen mit von Trägern/Matrizes *in situ* freizusetzenden (Wirk-)stoffen zur therapeutische Anwendung für die vorliegende Erfindung in Frage kommen, wobei dem Fachmann jeweils klar sein wird, dass der einzusetzende (Wirk-)Stoff bzw. die einzusetzenden Zellen vom jeweiligen zu behandelnden Fall abhängen.

In einer Ausführungsform der erfindungsgemäßen therapeutische Verwendung ist bevorzugt, wenn das Serumalbumin oder das Serumprotein durch Gruppen funktionalisiert ist, die aus Maleimid-, Vinylsulfon-, Acrylat-, Alkylhalogenid-, Azirin-, Pyridyl-, Thionitrobenzolsäuregruppen, oder arylierenden Gruppen ausgewählt sind.

Unter "funktionalisiert" bzw. funktionalisieren ist vorliegend jeder - abgeschlossene - Vorgang zu verstehen, mit dem dem Polymer - bspw. durch Hinzufügen von Gruppen an das Polymer - eine Funktion verliehen wird, die es normalerweise nicht besitzt.

Durch die Funktionalisierung des Polymers mit Maleimidgruppen kann eine gute Vernetzung des Polymers und gleichzeitig die Lebensfähigkeit von Zellen oder Biofunktionalität von Substanzen gewährleistet werden, wenn diese in die Zusammensetzung/das Material eingebracht werden. Die in die Zusammensetzung ggf. einzubringenden Zellen oder Substanzen werden durch Dispergierung in die Zusammensetzung mit dem funktionalisierten Polymer eingebracht, das mit den Zellen/Substanzen vernetzt.

Wie bereits weiter oben erwähnt betrifft die Erfindung auch die therapeutische Verwendung der Zusammensetzung oder des Materials zur Beschichtung und Oberflächenmodifikation von Implantaten, die aus anderen Materialien als das Material bestehen, das ausgehend von der genannten Zusammensetzung polymerisiert ist.

Eine solche Beschichtung bzw. Modifikation bietet die Möglichkeit, Implantate, die aus einem anderen, nicht vergleichbar verträglichem Material bestehen, zu beschichten, um so diese Implantate, die normalerweise die Endothelzell-Proliferation und damit auch eine Angiogenese fördern, non-angiogen zu machen. Dabei kommen jegliche Implantate in Betracht, insbesondere solche, die wiederum selber auf Hydrogelen basieren, die aber nicht auf der Zusammensetzung basieren. Dies ist ferner insbesondere in den Fällen vorteilhaft, wo eine direkte chemische Anbindungschemie, wie sie für das polymerisierte Material eingesetzt wird, möglich ist. Dies erlaubt eine kovalente Anbindung einer dünnen Materialschicht an das Implantatmaterial.

Wie bereits weiter oben erwähnt, betrifft die Erfindung auch die therapeutische Verwendung der beschriebenen Zusammensetzung oder des polymerisierten hydrogelbildenden Materials zur Behandlung oder Prävention von Angiogenese-assoziierten Krankheiten.

Diese Maßnahme hat den Vorteil, dass diese Krankheiten durch die erfindungsgemäße therapeutische Verwendung durch die Inhibierung der Angiogenese gelindert oder sogar präventiv verhindert werden können.

Eine Auflistung von Angiogenese-assoziierten Krankheiten ist bspw. in Carmeliet, "Angiogenesis in health and disease", Nature Medicine (2003), Vol. 9 Nr. 6: 653-660, zu finden, und insbesondere in der dort aufgeführten Tabelle 1, in der Krankheiten, welche durch übermäßige Angiogenese charakterisiert werden, aufgelistet sind. Bspw. zählen hierzu Krebs, einige Infektionskrankheiten, Autoimmunerkrankungen, DiGeorge Syndrom, Arteriosklerose, Fettsucht, Psoriasis, Karposi Sarkom, diabetische Retinopathie, primäre pulmonale Hypertension, Asthma bronchiale, peritoneale Adhäsionen, Endometriose, Arthristis, Synovitis, Osteophytenbildung, Osteomyelitis. Weitere Vorteile ergeben sich aus der Beschreibung und der beigefügten Zeichnung.

Es versteht sich, dass die vorstehend genannten und die nachstehend noch zu erläuternden Merkmale nicht nur in den jeweils angegebenen Kombinationen, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:

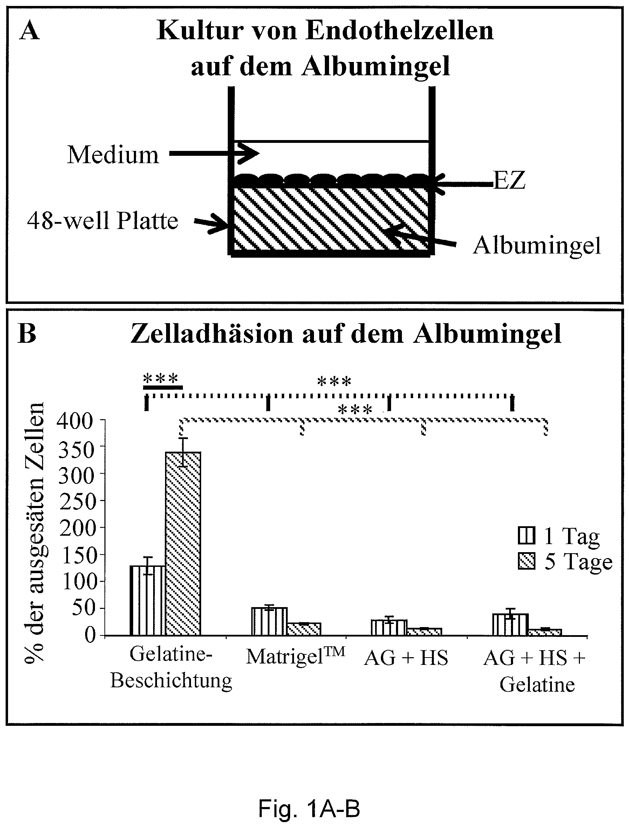

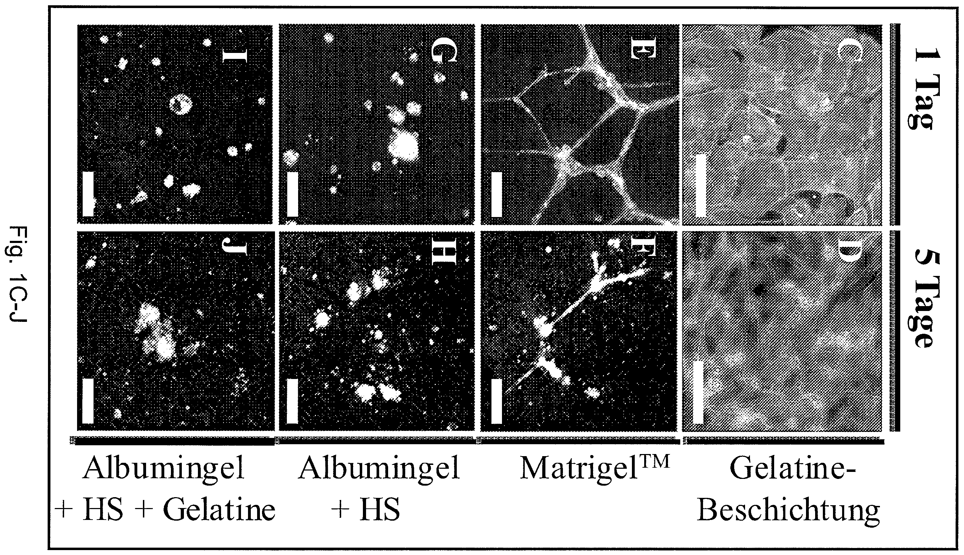

- Fig. 1: Die Ergebnisse zu Adhäsionsversuchen von Endothelzellen auf einem polymerisierten, hydrogelbildenden, auf Serumalbumin basierenden Material (im Folgenden auch "Albumingel" oder "Albugel"): Schematische Darstellung der Kultur von Endothelzellen auf dem Albugel (A); Diagramm zur Quantifizierung der Anzahl von Endothelzellen auf dem Albugel nach 1 Tag und 5 Tagen (B); Phalloidin-gefärbte Endothelzellen bei den verschiedenen Kulturbedingungen (C);

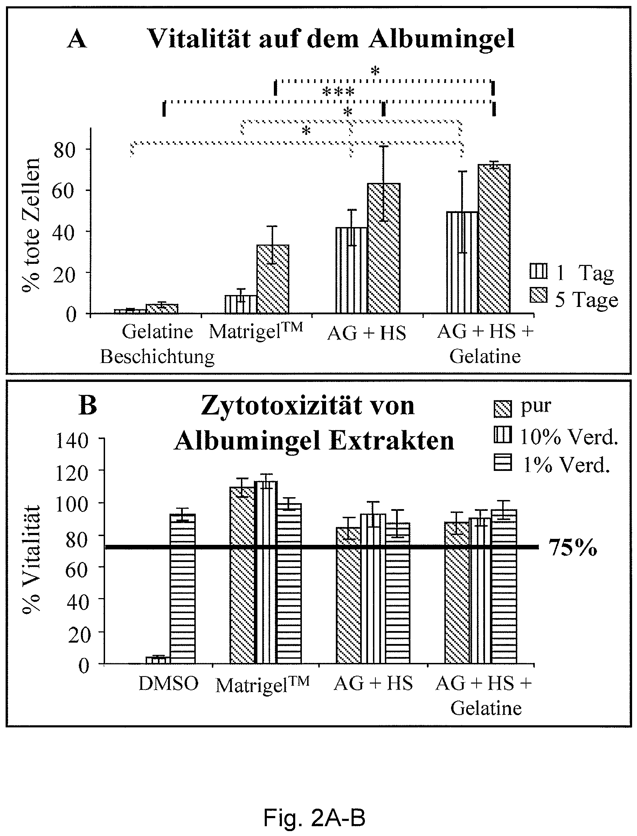

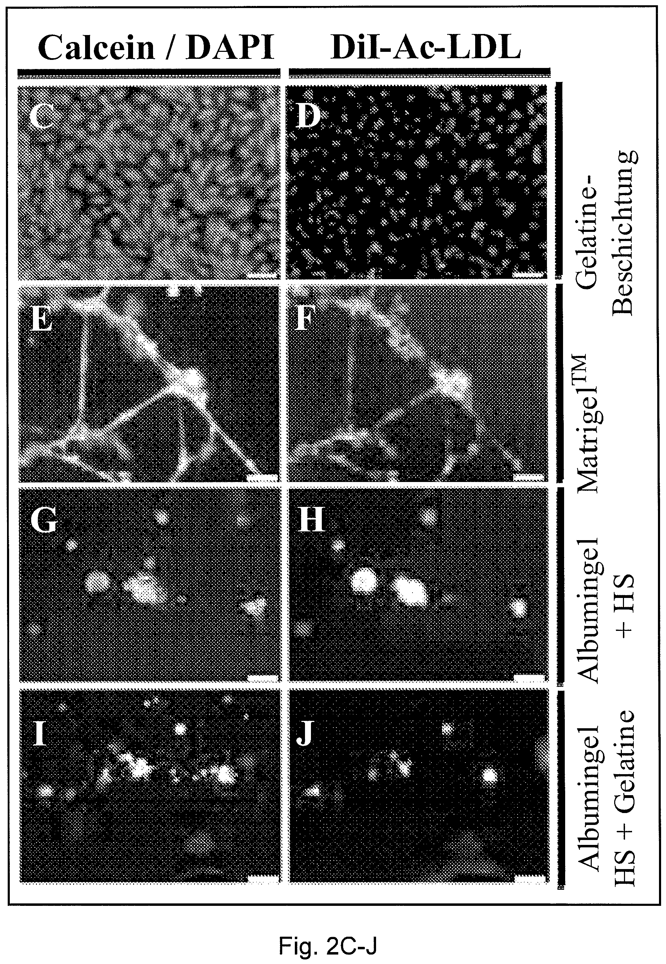

- Fig. 2: den Nachweis der Vitalität der Endothelzellen auf Albugel: Diagramm zur Quantifizierung von Endothelzellen auf dem Albugel (A); Diagramm zur Untersuchung der zytotoxischen Wirkung von Albugel-Extrakten auf Endothelzellen (B); Calcein- und DAPI-gefärbte Endothelzellen bei den verschiedenen Kulturbedingungen (C, E, G, I) und Aufnahme von DiI-Ac-LDL (D, F, H, J);

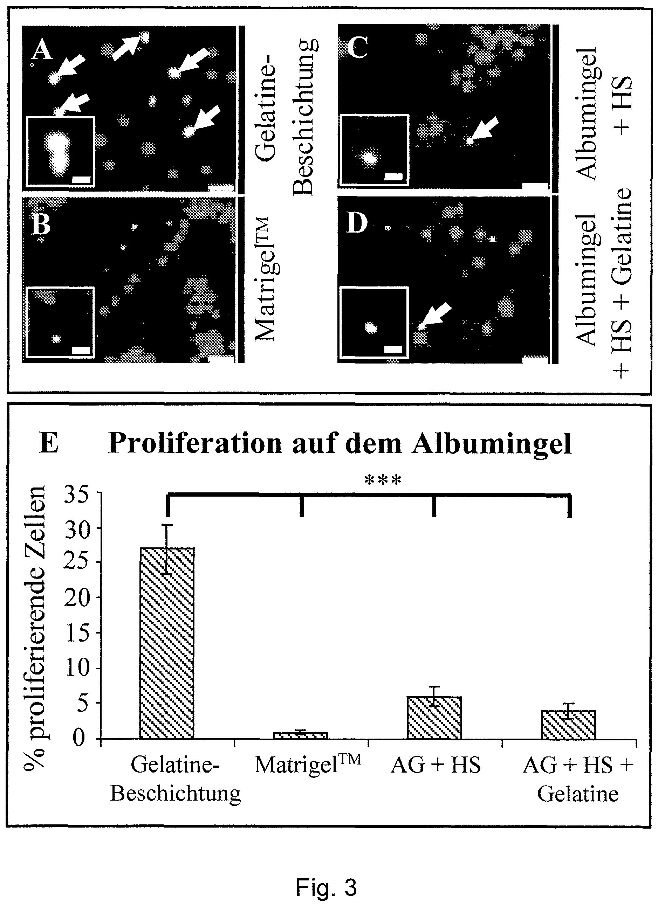

- Fig. 3: die Ergebnisse zur Untersuchung zur Proliferation von Endothelzellen auf Albugel: DAPI- und BrdU-gefärbte Endothelzellen bei den verschiedenen Kulturbedingungen (A-D); Diagramm zur Quantifizierung der Proliferation von Endothelzellen auf dem Albugel (E);

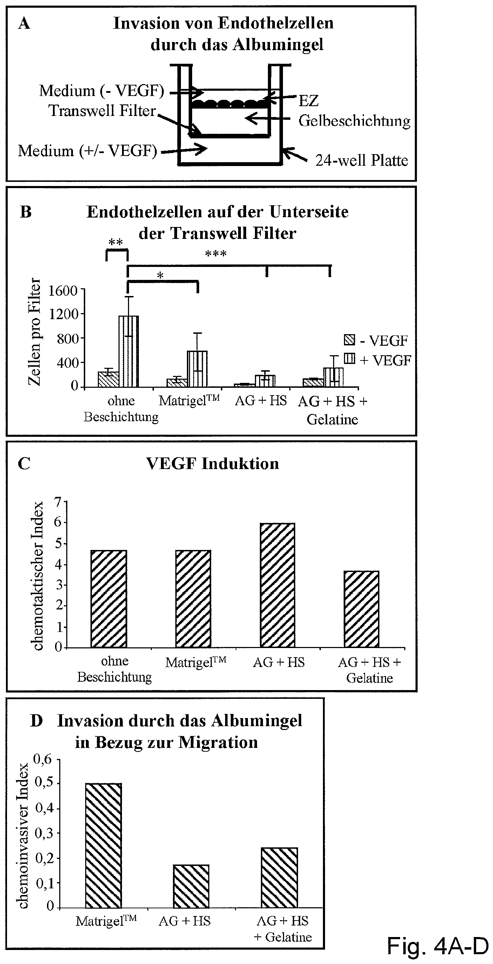

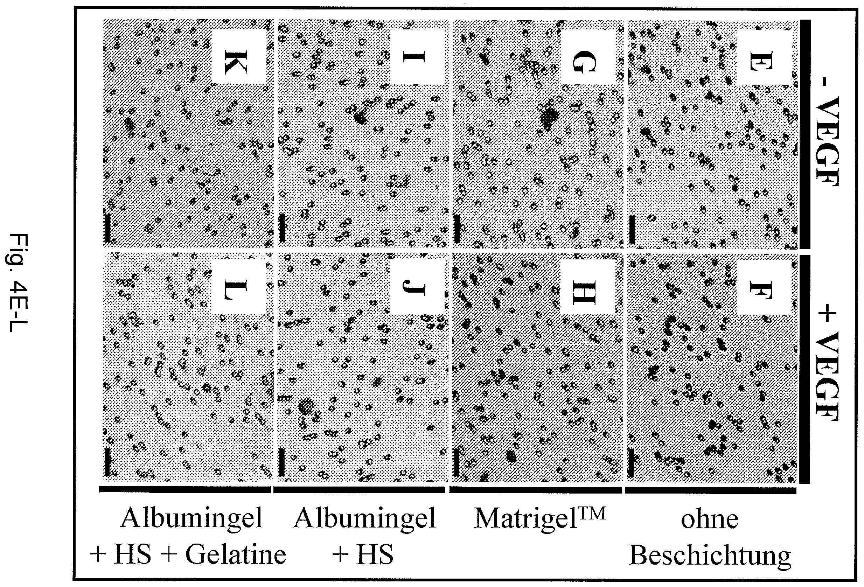

- Fig. 4: die Ergebnisse der Untersuchungen der Invasion von Endothelzellen durch das Albugel: schematische Darstellung des Aufbaus (A); Diagramm zur Quantifizierung von durch das Albumingel gewanderten Endothelzellen (B); Diagramm zur Analyse des chemotaktischen Index (C); Diagramm zur Analyse des chemoinvasiven Index (D), Rose-Bengal-gefärbte Endothelzellen auf der Unterseite der Transwell-Filter (E-L);

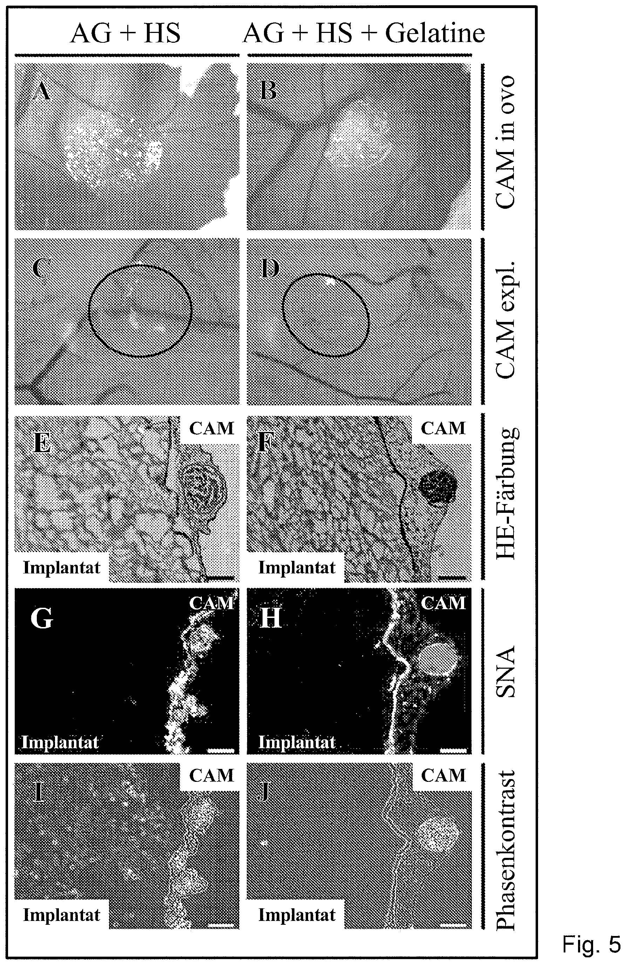

- Fig. 5: die Ergebnisse der Untersuchungen zum Einwachsen von Blutgefäßen der Chorioallantoismembran in das Albugel: Fotos der Implantate *in ovo* (A, B); Fotos der explantierten Chorioallantoismembran mit dem Albugel (C, D); HE- (Hämatoxylin-Eosin) gefärbte Chorioallantoismembran mit dem Albugel (E, F); Sambucus nigra Lektin-gefärbte Chorioallantoismembran mit Albugel (G, H); und Phasekontrastaufnahmen zu G und H (I, J);

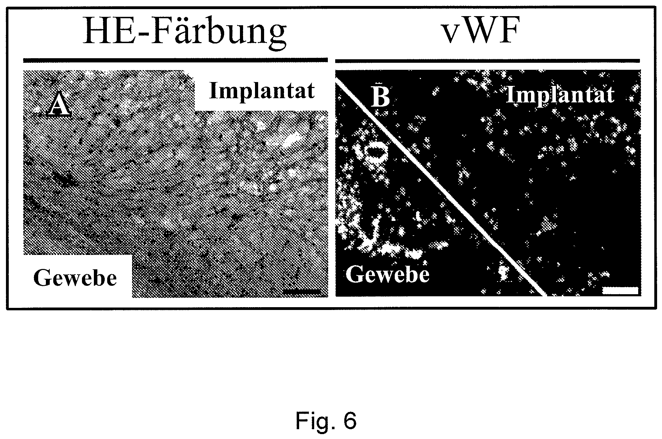

- Fig. 6: die Ergebnisse zu Implantationsversuchen von Albugel subkutan in den Rücken einer Skid/nu Maus: HE-Färbung des Albugels mit umgebendem Mausgewebe (A), Sambucus nigra Lektin-Färbung und DAPI Färbung des Albugels mit umgebendem Mausgewebe (B); und

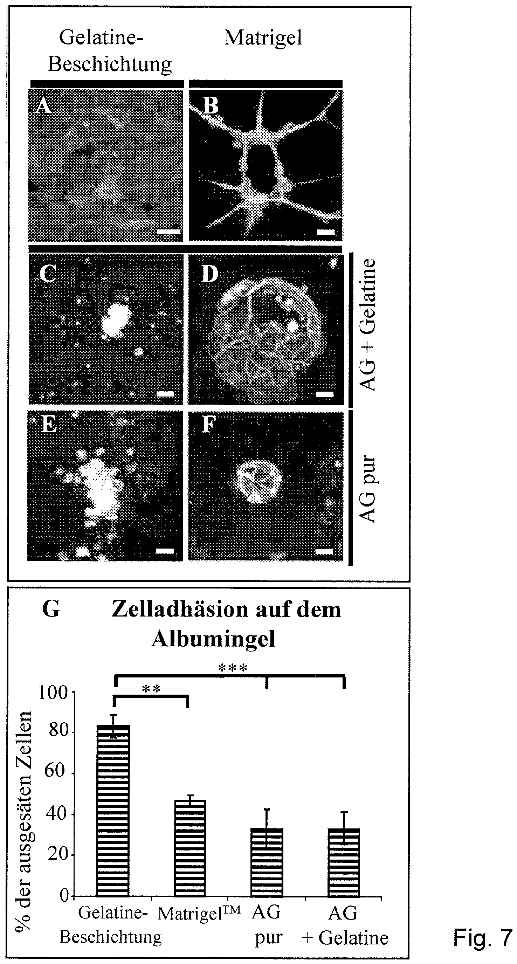

- Fig. 7: die Ergebnisse zu Adhäsionsversuchen von immortalisierten Endothelzellen auf dem Albugel: Phalloidin-gefärbte Endothelzellen bei den verschiedenen Kulturbedingungen (A-F); Diagramm zur Quantifizierung der Anzahl von Endothelzellen auf dem Albugel nach 1 Tag (G).

### Beispiele:

### A) Herstellung von maleimidmodifiziertem Serumalbumin

250 mg humanes, Kaninchen- oder Schaf-Serumalbumin (Sigma-Aldrich) wurden in 5 ml 1M Na-Borat (pH 8,2) gelöst. Dazu wurden 75 µl einer 260 mM N-Maleoyl-β-Alanin (Sigma-Aldrich Kat.-Nr 63285)-Lösung in PBS/Na-Borat (pH 8,2) (1:1) hinzugefügt, und für 90 min bei Raumtemperatur inkubiert. 106 mg 3-Maleimidopropionsäure-N-Hydroxysuccinimidester (SMP, Obiter Research, Urbana, IL, USA) in 950 µl Dimethylformamid (DMF) gelöst. Unlösliches Material wurde durch Zentrifugation abgetrennt. 500 µl des Überstandes wurden zu der Albuminlösung gegeben, die danach für 60 min bei Raumtemperatur inkubiert wurde. Danach wurden 500 µl 3M Natriumacetat (pH 4,7) dazu gegeben und dreimal gegen 1 Liter PBS auf Eis dialysiert. Das Dialysat wurde anschließend durch Ultrafiltration (YM-3 Membran, Millipore) auf ein Volumen von 3,5 ml konzentriert, filtersterilisiert und bei -80°C gelagert.

Das so funktionalisierte Serumalbumin/-protein kann durch Zugabe von SH-Vernetzern polymerisiert werden. Dabei kommen insbesondere der Vernetzer Bis-Thio Polyethylenglykol in Betracht, der an beiden Enden eine SH-Gruppe trägt. Neben Bis-Thio-PEG kommen als Vernetzer generell Substanzen in Betracht, die SH-Gruppen tragen, insbesondere Polymere, und bspw. Dithio-PEG oder SH-modifiziertes Dextran, SH-modifiziertes Polyvinylalkohol, SH-modifiziertes Polyvinylpyrrolidon, etc.

Bis-Thio-PEG ist kommerziell erhältlich, verwendet wurde der Vernetzer mit einer Molmasse von 10000 g/mol. Liegt die Molmasse darunter, verringert dies die Gelbildung, bei höheren Massen geliert das Gel zu schnell, was eine ausreichende Vermischung der Substanzen unmöglich macht. Die beste Gelbildung wird erreicht, wenn SH-Gruppen des Vernetzers und Maleimidgruppen des Albumins in äquimolaren Konzentrationen vorliegen. Verwendet wurde jeweils eine Endkonzentration von 3 mM Maleimid und SH-Gruppen im Gel. Zusätzlich enthielt das Schaf-Albugel 4 mg/ml hochpolymere Hyaluronsäure (im Folgenden und in den Figuren auch mit "HS" bezeichnet/abgekürzt), die vor Polymerisation zugemischt wird und daher physikalisch fest verankert vorliegt. Als Albuminquelle sind aber die verschiedensten tierischen und menschlichen Serumalbumine verwendbar.

### B) Versuche zum Nachweis der non-angiogenen Eigenschaft des Albugels

### 1. Test des Albugels als Substrat für humane Endothelzellen

a) Um die Wirkungen von Albugel auf Endothelzellen ("EZ") zu untersuchen, wurden primäre humane Endothelzellen aus der Nabelschnurvene (HUVEC) (PromoCel, Heidelbergl) der Passagen 3 bis 9 auf Gel kultiviert und anschließend die Adhäsion, die Vitalität und die Proliferation der Zellen untersucht. Hierzu wurden je 100 µl Schaf-Albugel in einer 48-well Platte auspolymerisiert und je 1,5 × 10⁴ HUVECs in 300 µl Endothelzellmedium pro well für 24 Stunden oder für 5 Tage auf dem Gel kultiviert (Versuchsaufbau Siehe Fig. 1A). Zur Kontrolle wurde die gleiche Anzahl Zellen in einer gelatinierten (0,5%) 48-well Platte kultiviert und ein Albugel mit zusätzlich 0,5% Gelatine (Endkonzentration in dem Gel) hergestellt. Gleichzeitig wurden die Zellen auf 10 mg/ml Matrigel^{™} kultiviert, eines wenig definierten Basalmembranextraktes aus dem Engelbreth-Holm-Swarm Maus-Sarkom, der als Basalmembranäquivalent in der Grundlagenforschung fungiert.
**Herstellung der Albugele** (im Folgenden und in den Figuren auch mit "AG" abgekürzt/bezeichnet):

| | Schafserumalbumingel mit Hyaluronsäure |
|---|---|
| Endothelzellmedium (ohne FCS) mischen mit | X x 53 µl |
| maleimidmodifiziertem Schafersumalbumin und | X x 7 µl |
| Visiol (20 mg/ml) | X x 20 µl |
| Bis-Thio-PEG vorlegen in Patte und mit Gelmaterial mischen | X x 20 µl |
| **Endvolumen** | **X x 100 µl** |

**Gelatinebschichtung und Matrigel^{™}:**
Für die Gelatinebeschichtung wurden 2% Gelatinelösung 1:4 mit PBS (Phosphat-gepufferter Saline) gemischt und die Platten damit für 30 min. inkubiert. Anschließend wurden die Platten einmal mit PBS gewaschen. Matrigel^{™} (20 mg/ml; BD Biosciences, San Jose, USA) wurde 1:2 mit Endothelzellmedium ohne FCS (fetales Kälberserum) gemischt und für 20 min bei 37°C in der Platte auspolymerisiert.
Die Zellen wurden für 1 Tag oder für 5 Tage bei den verschiedenen Kultivierungsbedingungen kultiviert. Zur Auswertung wurden die Endothelzellen wie folgt behandelt:
   Nach 1 Tag und nach 5 Tagen wurden die HUVEC zur Bestimmung der Zelladhäsion mit 2,5% Glutaraldehyd/PBS fixiert, mit 0,2% Triton-X 100/PBS permeabilisiert und anschließend mit DAPI (4',-Diamidino-2-phenylindol) und Phalloidin Oregon Green gefärbt.
   Oder: Mithilfe des "5-Bromo-2'-deoxy-Uridin Cell Labeling and Detetion Kit I" (Roche, Mannheim) wurden proliferierende HUVEC nach 1 Tag sichtbar gemacht.
   Oder: Nach 1 Tag und 5 Tagen wurden die Zellen mit DiI-Ac-LDL (1,1'-Dioctadecyl-3,3,3',3'-tetramethyl-indocarbocyanin Perchlorat-acetyliertes Low-Density-Lipoprotein) behandelt und anschließend mit Calcein und DAPI zur Detektion vitaler und toter HUVEC gefärbt.

b) Zusätzlich wurden Albugel-Extrakte hinsichtlich ihrer zytotoxischen Wirkung auf Endothelzellen untersucht.
Herstellung der Extrakte:
200 µl Albugel (mit und ohne Gelatinezusatz) wurden in einem Eppendorf-Gefäß auspolymerisiert und für 24 Stunden mit 1 ml Endothelzellmedium bei 37°C schüttelnd kultiviert. Zur Kontrolle wurden Extrakte von Matrigel^{™} entsprechend hergestellt. Die Endothelzellen wurden für 24 Stunden mit den verschiedenen Extrakten und mit DMSO (Dimethylsulfoxid) als Totkontrolle inkubiert. Die Vitalität der Zellen, in Bezug gesetzt zu Zellen, welche lediglich mit Medium kultiviert wurden, wurde mittels Alamar Blue Assay ermittelt.
c) Ergebnisse:
Wie Fig. 1 entnommen werden kann, lag nach 1 Tag Kultur auf den Albugelen und auf Matrigel^{™} die Zellzahl deutlich unter der Zellzahl der Gelatinebeschichtung. Nach 5-tägiger Kultur sank die Zellzahl auf den Gelen, wohingegen sie auf Gelatine weiter stieg. Die Morphologie der Zellen bei den verschiedenen Kulturbedingungen kann den Fig. 1 C-J entnommen werden. Während die Endothelzellen auf den Albugelen Aggregate bildeten und nicht auf dem Gel adhärieren konnten, breiteten sich die Endothelzellen auf Gelatine aus und bildeten auf Matrigel^{™} typische "Tubes" aus. Die Ergebnisse zeigen, dass Endothelzellen nicht auf dem Albugel adhärieren können und z.B. bei Mediumwechsel von dem Gel gelöst werden.
Die Vitalität der Endothelzellen ist in der Fig. 2A und qualitativ in der Fig. 2C, E, G und I dargestellt. Während auf der Gelatinebeschichtung auch nach 5 Tagen kaum tote Zellen zu detektieren waren und auch auf Matrigel^{™} nach 5 Tagen weniger als 40% der Zellen tot waren, stieg die Anzahl toter Endothelzellen auf den Albugelen auf über 60% an. Hingegen waren vitale Zellen bei allen Kulturbedingungen in der Lage DiI-Ac-LDL aufzunehmen (siehe Fig. 2D, F, H, J), die Funktionalität der Endothelzellen bleibt demnach erhalten. Extrakte der Albugele wiesen zusätzlich keine zytotoxische Wirkung auf die Endothelzellen auf (Fig. 2, B).
Wie der Fig. 3 (A-D qualitativ, E quantitativ) entnommen werden kann, proliferierten Endothelzellen auf dem Albugel im Gegensatz zur Gelatinebeschichtung nur in einem geringen Maße.
Die Zugabe von Gelatine, die für ihre proangiogenen und proadhäsiven Eigenschaften bekannt ist, in das Albumingel zeigte keinen positiven Effekt auf die Endothelzellen.

### 2. Test des Albugels als Substrat für humane Endothelzellen

Um auszuschließen, dass Hyaluronsäure ("HS") für die beobachteten Effekte verantwortlich ist, wurden im Weiteren Versuche mit Kaninchen-Albugel ohne Hyaluronsäure durchgeführt.
a) Durchführung:
Für die Kultur wurden immortalisierte humane Endothelzellen aus der Nabelschnurvene (HUVEC hTERT) verwendet.
1,5×10⁴ HUVEC wurden in 300 µl Endothelzellmedium auf 0,5% Gelatinebeschichtung, 100 µl Matrigel^{™} (10 mg/ml) oder 100 µl Albumingel pur in einer 48-well Platte kultiviert. Als weitere Kontrolle wurde zusätzlich mit 0,5% Gelatine (Endkonzentration) hergestellt.
Herstellung der Albumingele:

| | Kaninchenserumalbumingel |
|---|---|
| Endothelzellmedium (ohne FCS) mischen mit | X x 73 µl |
| maleimidmodifiziertem Schafserumalbumin und | X x7 µl |
| Bis-Thio-PEG vorlegen in Platte und mit Gelmaterial mischen | X x 20 µl |
| Endvolumen | X x 100 µl |

Gelatinebeschichtung und Matrigel^{™}:
Für die Gelatinebeschichtung wurden 2% Gelatinelösung 1:4 mit PBS (Phosphate gepufferte Saline) gemischt und die Platten damit für 30 min inkubiert. Anschließend wurden die Platten einmal mit PBS gewaschen.
Matrigel^{™} (20 mg/ml) wurde 1:2 mit Endothelzellmedium ohne FCS (fetal Kälberserum) gemischt und für 30 min bei 37°C in der Platte auspolymerisiert. Die Zellen wurden für 1 Tag bei den verschiedenen Kulturbedingungen kultiviert.
Zur Auswertung wurden die Endothelzellen wie folgt behandelt:
   Nach 1 Tag wurden die HUVEC zur Bestimmung der Zelladhäsion mit 2,5% Glutaraldehyd/PBS fixiert, mit 0,2% Triton-X 100/PBS permeabilisiert und anschließend mit DAPI (4',6-Diamidino-2-phenylindol) und Phalloidin Oregon Green gefärbt.

b) Ergebnisse:
Wie der Fig. 7G entnommen werden kann, liegt nach 1 Tag Kultur auf den Albugelen und auf Matrigel^{™} die Zellzahl deutlich unter der Zellzahl der Gelatinebeschichtung. Die Morphologie der Zellen bei den verschiedenen Kulturbedingungen kann der Fig. 7 A - H entnommen werden. Die Endothelzellen breiten sich auf Gelatine aus und bilden auf Matrigel^{™} typische "Tubes" aus. Endothelzellen auf dem Albugel bilden Aggregate (Abb. 7C und E) oder sie bilden eine Art Sphäroidstruktur aus (Abb. 7D und F).

### 3. Invasion von Endothelzellen durch das Albugel

a) Die Invasion von Endothelzellen durch das Albugel auf einem Transwell-Filter wurde verglichen zur Invasion durch Matrigel^{™} und der Migration durch einen unbeschichteten Filter. Eine schematische Darstellung des Versuchsaufbaus kann der Fig. 4A entnommen werden.
   Transwell-Filter mit einer Porengröße von 8 µm wurden mit 100 µl Albugel mit Hyaluronsäure, 100 µl Albuminsäure mit 0,5% Gelatine und 100 µl Matrigel^{™} (5 mg/ml) beschichtet. Zur Bestimmung der Migration wurden unbeschichtete Transwell-Filter verwendet.
   Pro Ansatz wurden 3×10⁵ Hoechst 33258-markierte HUVEC in 200 µl Endothelzellmedium auf die Filter überführt. Nachdem sich die Zellen für 2 Stunden abgesetzt hatten, wurden 600 µl Endothelzellmedium mit und ohne 40 ng/ml VEGF (Vascular endothelial growth factor) in das untere Kompartiment pipettiert. Nach 24 Stunden wurden die Zellen auf der Oberseite des Filters abgewischt und die Zellen auf de Unterseite des Filters fixiert und ausgezählt. Alternativ wurden die Zellen mit Rose Bengal gefärbt.
b) **Ergebnisse:**
   Die Anzahl der Endothelzellen auf der Unterseite der Transwell-Filter ist in Fig. 4B gezeigt, Rose Bengal gefärbte Endothelzellen in Fig. 4E-L. Aus den Mittelwerten der Anzahl der Zellen auf der Unterseite der Filter wurde der chemotaktische Index (siehe Fig. 4C), der den Quotienten aus Migration oder Invasion mit VEGF Induktion zu ohne VEGF Induktion angibt und der chemoinvasive Index (siehe Fig. 4D), der den Quotienten aus durch ein Gel gewanderten und migrierten Zellen angibt, berechnet. Die chemotaktischen Indizes bei allen Beschichtungen lagen etwa gleich hoch, die Induktion durch VEGF ist demnach vergleichbar. Die chemoinvasiven Indizes der beiden Albugele lagen allerdings deutlich unter dem chemoinvasiven Index von Matrigel^{™}, was zeigt, dass Endothelzellen nur in geringem Maße durch das Albugel wandern können.

### 4. Einwachsen von Blutgefäßen der Chorioallantoismembran in das Albugel

a) Durchführung
   In Eier der Rasse Hissex Braun wurde an Embryonaltag 3 ein Fenster in die Eischale eingefügt. Am Embryonaltag 8 wurden 200 µl Albugel mit Hyaluronsäure und 200 µl Albugel mit Hyaluronsäure und 0,5% Gelatine auf die stark vaskularisierte Chorioallantoismembran (CAM) überführt. Die Eier wurden bis Embryonaltag 13 weiter inkubiert. Die CAM wurde in 4% PFA (Paraformaldehyd)/PBS bei Raumtemperatur *in ovo* fixiert, anschließend explantiert und für einen weiteren Tag bei 4°C fixiert, für 2 Tage in 30% Saccharose/destilliertem Wasser entwässert und in Tissue Tek (O.C.T. Compound, Sakura; Torrance Kanada) eingefroren. 7 µm dicke Gefrierschnitte wurden HE- (Hämatoxylin-Eosin) und 5 µm dicke Gefrierschnitte mit dem Sambucus nigra Lektin gefärbt.
b) Ergebnisse:
   Die Blutgefäße der CAM wachsen nicht auf die implantierten Albugele zu (Fig. 5 A-D). Weder in HE gefärbten (Fig. 5 E und F) noch in Sambucus nigra Lektin gefärbten Schnitten (Fig. 5 G und H) konnten Blutgefäße nachgewiesen werden, wobei Blutgefäße in der CAM mithilfe beider Färbemethoden detektiert werden konnten. Die Ergebnisse zeigen, dass das Albugel keinen angiogenen Einfluss auf die Blutgefäße der CAM ausübte.

### 5. Implantation des Albugels in den Rücken einer Scid/nu Maus

a) Albumingele, basierend auf humanem Serumalbumin wurden mit humanen Bandscheibenzellen besiedelt und in den Rücken von Scid/nu Mäusen injiziert. Zwei Wochen nach dem Implantation wurden die Albumingele wieder explantiert und Schnitte dieser explantierten Albumingele nach HE-Färbung untersucht. Zusätzlich wurden Blutgefäße mittels einer immunhistochemischen Färbung gegen den humanen von Willebrand Faktor detektiert.
b) Ergebnisse
   Mittels HE-Färbung konnten weder im umgebenden Mausgewebe noch in den Implantaten Blutgefäße nachgewiesen werden (Fig. 6A), die Zellkerne humaner Bandscheibenzellen in dem Implantat wurden durch das Hämatoxylin gefärbt. Die spezifische Färbung von Blutgefäßen mit einem Antikörper gegen den humanen von Willebrand Faktor zeigte, dass sich in dem umgebenden Gewebe Blutgefäße befanden, nicht jedoch in das Albugel einwuchsen (Fig. 6B). In dem Implantat sind lediglich die DAPI-gefärbten humanen Bandscheibenzellen erkennbar.

Zusammenfassend konnte mit den oben beschriebenen Versuchen gezeigt werden, dass Endothelzellen auf Albugel kaum adhärieren oder proliferieren. Darüber hinaus wurde gezeigt, dass Endothelzellen auf dem Albugel absterben, und zwar nicht etwa aufgrund einer Toxizität des Albugels, sondern vielmehr aufgrund der fehlenden überlebenswichtigen Zelladhäsion. Auch konnte durch Zugabe des chemotaktischen Lockstoffs VEGF nicht eine Wanderung der Endothelzellen in das Albugel erreicht werden, und auch Blutgefäße der Hühnerei-Chorioalllantoismembran wandern nicht in das Albugel ein. Auch *in vivo* Versuche an der Maus mit dem Albugel zeigten kein Einwandern von Blutgefäßen in das Albugel.

Diese non-permissiven Eigenschaften für Endothelzellen bieten somit die Möglichkeit der Verwendung des Albugels als Matrix/Implantat zur Inhibierung und Prävention der Angiogenese und der Adhäsion von Endothelzellen, insbesondere im Implantationsbereich der Medizin, bspw. bei der Behandlung von Sklerosen, und der regenerativen Medizin, bspw. bei der Behandlung von erkranktem und/oder defizientem Knorpel-, Bandscheiben-, Corneagewebe.

## Patentansprüche

1. Biokompatible Zusammensetzung, die zu einem hydrogelbildenden Material polymerisierbar ist und die auf einem hydrophilen Polymer basiert, wobei das hydrophile Polymer vernetzbares Serumalbumin oder vernetzbares Serumprotein ist, zur therapeutischen Verwendung in der Inhibierung und/oder Prävention der Angiogenese oder Endothelzell-Proliferation.

2. Biokompatible Zusammensetzung zur therapeutischen Verwendung nach Anspruch 1, **dadurch gekennzeichnet, dass** die Zusammensetzung zumindest eines der Folgenden aufweist, Säugerzellen, ein pharmakologisches Agens, ein biologisch wirksames Agens, oder eins oder mehrere oder Mischungen davon.

3. Biokompatible Zusammensetzung zur therapeutischen Verwendung nach einem der vorstehenden Ansprüche, **dadurch gekennzeichnet, dass** das Serumalbumin oder das Serumprotein durch Gruppen funktionalisiert ist, die aus Maleimid-, Vinylsulfon-, Acrylat-, Alkylhalogenid-, Azirin-, Pyridyl-, Thionitrobenzolsäuregruppen, oder arylierenden Gruppen ausgewählt sind.

4. Biokompatible Zusammensetzung zur therapeutischen Verwendung nach einem der vorstehenden Ansprüche, **dadurch gekennzeichnet, dass** das Serumalbumin und das Serumprotein humanes Serumalbumin und humanes Serumprotein ist.

5. Biokompatible Zusammensetzung zur therapeutischen Verwendung nach einem der Ansprüche 2 bis 4, **dadurch gekennzeichnet, dass** das pharmakologisch wirksame Agens ausgewählt ist aus zumindest einem der Folgenden: einem Antibiotikum, einem entzündungshemmendem Mittel, einem Stoffwechsel-Hormon, Chondroprotektiva, Agentien zur Gentherapie, Wachstumshormone, Differenzierungs- oder Modulationsfaktoren, Immunsuppresiva, immun-stimulierende Substanzen, DMOAs, Nucleinsäuren, Apoptose-induzierende Wirkstoffe, adhäsionsvermittelnde Wirkstoffe, Rezeptoragonisten- und Rezeptorantagonisten, oder Mischungen davon.

6. Biokompatible Zusammensetzung zur therapeutischen Verwendung nach einem der vorstehenden Ansprüche, **dadurch gekennzeichnet, dass** die Albuminkonzentration in dem Material von ca. 5 bis ca. 15 mg/ml Material, insbesondere von ca. 10 mg/ml Material beträgt.

7. Biokompatible Zusammensetzung zur therapeutischen Verwendung nach einem der vorstehenden Ansprüche, **dadurch gekennzeichnet, dass** die Zusammensetzung in injizierbarer Form eingesetzt wird.

8. Biokompatible Zusammensetzung eines polymerisierten, hydrogelbildenden Materials, das durch Polymerisierung einer auf Serumalbumin oder Serumproteinen basierenden Zusammensetzung erhalten wurde, zur therapeutischen Verwendung in der Inhibierung und/oder Prävention der Angiogenese oder Endothelzell-Proliferation.

9. Biokompatible Zusammensetzung zur therapeutischen Verwendung nach Anspruch 8, **dadurch gekennzeichnet, dass** das Material als Implantat eingesetzt wird.

10. Biokompatible Zusammensetzung zur therapeutischen Verwendung nach einem der Ansprüche 1 bis 6, **dadurch gekennzeichnet, dass** die Zusammensetzung als Oberflächenbeschichtung eines Implantates eingesetzt wird.

11. Biokompatible Zusammensetzung zur therapeutischen Verwendung nach Anspruch 8, **dadurch gekennzeichnet, dass** das Material als Oberflächenbeschichtung eines Implantates eingesetzt wird.

## Claims

1. A biocompatible composition, which can be polymerized to give a hydrogel-forming material and which is based on a hydrophilic polymer, wherein the hydrophilic polymer is crosslinkable serum albumin or crosslinkable serum protein, for therapeutic use in inhibiting and/or preventing angiogenesis or endothelial cell proliferation.

2. The biocompatible composition for therapeutic use as claimed in claim 1, **characterized in that** the composition comprises at least one of the following: mammalian cells, a pharmacological agent, a biologically active agent, or one or more or mixtures thereof.

3. The biocompatible composition for therapeutic use as claimed in one of the preceding claims, **characterized in that** the serum albumin or the serum protein is functionalized by groups, which are selected from among maleimide, vinylsulfonic, acrylate, alkyl halide, azirine, pyridyl, thionitrobenzene acid groups or arylating groups.

4. The biocompatible composition for therapeutic use as claimed in one of the preceding claims, **characterized in that** the serum albumin and the serum protein are human serum albumin and human serum protein.

5. The biocompatible composition for therapeutic use as claimed in any of claims 2 to 4, **characterized in that** the pharmacologically active agent is selected from at least one of the following: an antibiotic, an anti-inflammatory, a metabolism hormone, chondroprotectants, agents for gene therapy, growth hormones, differentiation or modulation factors, immunosuppressants, immuno-stimulants, DMOAs, nucleic acids, apoptosis-inducing active substances, adhesion-mediating active substances, receptor agonists and receptor antagonists, or mixtures thereof.

6. The biocompatible composition for therapeutic use as claimed in one of the preceding claims, **characterized in that** the albumin concentration in the material is from approximately 5 to approximately 15 mg/ml material, in particular from approximately 10 mg/ml material.

7. The biocompatible composition for therapeutic use as claimed in one of the preceding claims, **characterized in that** the composition is employed in injectable form.

8. A biocompatible composition of a polymerized hydrogel-forming material, which has been obtained by polymerizing a serum-albumin-based or serum-protein-based composition, for therapeutic use in inhibiting and/or preventing angiogenesis or endothelial cell proliferation.

9. The biocompatible composition for therapeutic use as claimed in claim 8, **characterized in that** the material is employed as an implant.

10. The biocompatible composition for therapeutic use as claimed in any of claims 1 to 6, **characterized in that** the composition is employed as surface coating of an implant.

11. The biocompatible composition for therapeutic use as claimed in claim 8, **characterized in that** the material is employed as surface coating of an implant.

## Revendications

1. Composition biocompatible, qui est polymérisable en un matériau formant un hydrogel, et qui est à base d'un polymère hydrophile, le polymère hydrophile étant une sérumalbumine réticulable ou une protéine du sérum réticulable, pour l'utilisation thérapeutique dans l'inhibition et/ou la prévention de l'angiogenèse et/ou de la prolifération des cellules endothéliales.

2. Composition biocompatible pour l'utilisation thérapeutique selon la revendication 1, **caractérisée en ce que** la composition comprend au moins l'un des éléments suivants : des cellules mammaliennes, un agent pharmacologique, un agent biologiquement actif ou un ou plusieurs ou des mélanges de ceux-ci.

3. Composition biocompatible pour l'utilisation thérapeutique selon l'une des revendications précédentes, **caractérisée en ce que** la sérumalbumine ou la protéine du sérum est fonctionnalisée par des groupes qui sont choisis parmi les groupes maléimide, vinylsulfone, acrylate, halogénure d'alkyle, azirine, pyridyle, acide thionitrobenzoïque, ou les groupes arylants.

4. Composition biocompatible pour l'utilisation thérapeutique selon l'une des revendications précédentes, **caractérisée en ce que** la sérumalbumine et la protéine du sérum sont la sérumalbumine humaine et la protéine du sérum humaine.

5. Composition biocompatible pour l'utilisation thérapeutique selon l'une des revendications 2 à 4, **caractérisée en ce que** l'agent pharmacologiquement actif est choisi parmi au moins l'un des éléments suivants : un antibiotique, un agent anti-inflammatoire, une hormone métabolique, des chondroprotecteurs, des agents de thérapie génique, des hormones de croissance, des facteurs de différenciation ou de modulation, des immunosuppresseurs, des substances immunostimulatrices, des DMOA, des acides nucléiques, des principes actifs inducteurs d'apoptose, des principes actifs promoteurs d'adhésion, des agonistes et des antagonistes de récepteurs ou des mélanges de ceux-ci.

6. Composition biocompatible pour l'utilisation thérapeutique selon l'une des revendications précédentes, **caractérisée en ce que** la concentration de l'albumine dans le matériau est d'environ 5 à environ 15 mg/ml de matériau, en particulier d'environ 10 mg/ml de matériau.

7. Composition biocompatible pour l'utilisation thérapeutique selon l'une des revendications précédentes, **caractérisée en ce que** la composition est utilisée sous une forme injectable.

8. Composition biocompatible d'un matériau polymérisé formant un hydrogel, qui a été obtenue par polymérisation d'une composition à base de sérumalbumine ou de protéine du sérum, pour l'utilisation thérapeutique dans l'inhibition et/ou la prévention de l'angiogenèse ou de la prolifération des cellules endothéliales.

9. Composition biocompatible pour l'utilisation thérapeutique selon la revendication 8, **caractérisée en ce que** le matériau est utilisé sous forme d'un implant.

10. Composition biocompatible pour l'utilisation thérapeutique selon l'une des revendications 1 à 6, **caractérisée en ce que** la composition est utilisée en tant que revêtement de surface d'un implant.

11. Composition biocompatible pour l'utilisation thérapeutique selon la revendication 8, **caractérisée en ce que** le matériau est utilisé en tant que revêtement de surface d'un implant.
